# FASCICULE DE BREVET EUROPEEN

(11) **EP 2 732 827 B1**
(45) Date de publication et mention de la délivrance du brevet: **16.09.2015**
(21) Numéro de dépôt: 13193625.4
(22) Date de dépôt: 20.11.2013
(51) Int. Cl.: A61L 2/07, A61L 2/00, G01N 33/68, G01N 33/50

(54) **Procédé de détermination de l'aptitude d'une formulation à inactiver un ATNC**
Verfahren zur Bestimmung der Fähigkeit einer Formulierung nichtkonventionell übertragbare Krankheitserreger zu inaktivieren
Method for determining the capability of a formulation to inactivate an non-conventional transmissible agent (NCTA)

(30) Priorité: 20.11.2012 FR 1260997
(43) Date de publication de la demande: 21.05.2014
(73) Titulaire: Franklab, 78180 Montigny le Bretonneux (FR)
(72) Inventeur: Fichet, Guillaume, 75016 Paris (FR)
(74) Mandataire: Nony

(56) Documents cités:
- UNAL ET AL: "Investigation by bioassay of the efficacy of sodium hydroxide treatment on the inactivation of mouse-adapted scrapie", BIOLOGICALS, ACADEMIC PRESS LTD., LONDON, GB, vol. 35, no. 3, 1 juin 2007 (2007-06-01), pages 161-164, XP022360752, ISSN: 1045-1056, DOI: 10.1016/J.BIOLOGICALS.2006.08.003
- EDGEWORTH JULIE ANN ET AL: "A standardized comparison of commercially available prion decontamination reagents using the Standard Steel-Binding Assay", JOURNAL OF GENERAL VIROLOGY,, vol. 92, no. 3, 1 mars 2011 (2011-03-01), pages 718-726, XP009156067, ISSN: 1465-2099
- SOLASSOL J ET AL: "DETECTION OF PRION AFTER DECONTAMINATION PROCEDURES: COMPARATIVE STUDY OF STANDARD WESTERN BLOT, FILTER RETENTION AND SCRAPIE-CELL ASSAY", JOURNAL OF HOSPITAL INFECTION, ACADEMIC PRESS, LONDON, GB, vol. 57, no. 2, 12 mai 2004 (2004-05-12), - 1 juin 2004 (2004-06-01), pages 156-161, XP009049138, ISSN: 0195-6701, DOI: 10.1016/J.JHIN.2004.03.005
- FICHET G ET AL: "Novel methods for disinfection of prion-contaminated medical devices", THE LANCET, LANCET LIMITED. LONDON, GB, vol. 364, no. 9433, 7 août 2004 (2004-08-07), pages 521-526, XP004738179, ISSN: 0140-6736, DOI: 10.1016/S0140-6736(04)16810-4
- FICHET ET AL: "Investigations of a prion infectivity assay to evaluate methods of decontamination", JOURNAL OF MICROBIOLOGICAL METHODS, ELSEVIER, AMSTERDAM, NL, vol. 70, no. 3, 25 août 2007 (2007-08-25), pages 511-518, XP022212060, ISSN: 0167-7012, DOI: 10.1016/J.MIMET.2007.06.005

## Description

### DOMAINE DE L'INVENTION

La présente invention se rapporte au domaine de la stérilisation, et en particulier au domaine de l'inactivation des agents transmissibles non conventionnels (ATNC) tel que la forme pathologique du prion PrP^{sc} ou PrP^{res}. L'invention se rapporte en particulier à une méthode de détermination de l'aptitude de substances ou compositions à inactiver un ATNC.

### ART ANTERIEUR

Les maladies provoquées par les agents transmissibles non conventionnels (ATNC) ont des caractères spécifiques communs (i) elles présentent des symptômes neurologiques, (ii) elles sont toujours mortelles, (iii) elles ont une période d'incubation très longue, (iv) l'agent causal résiste à la plupart des méthodes conventionnelles d'inactivation, et (v) l'agent franchit la barrière des espèces.

Les encéphalopathies spongiformes subaigües transmissibles (ESST) ou maladie à prions sont des maladies neurodégénératives qui affectent aussi bien l'homme que l'animal. L'agent infectieux à l'origine de ces maladies, le prion, serait de nature protéique. Les ESST ne sont pas des maladies nouvelles. La tremblante du mouton est connue depuis le 18ème siècle et la maladie de Creutzfeldt-Jakob (MCJ) depuis le siècle dernier. En revanche, l'épizootie d'encéphalopathie spongiforme bovine (ESB) a débuté en 1986 et le passage de l'agent bovin à l'Homme à l'origine de la variante de la maladie de Creutzfeldt-Jakob (vMCJ) a été rapporté en 1996. Les dernières estimations dénombrent à moins de 4000 individus potentiellement en phase d'incubation de la vMCJ (Ghani *et al*., 2003) et pourraient être alors la source de nouvelles contaminations interhumaines aussi bien via la transfusion sanguine que le matériel médico-chirurgical.

Les prions sont plutôt des agents hydrophobes. Cette caractéristique leur confère une grande capacité d'adsorption sur des surfaces solides très diversifiées. La contamination de surface est beaucoup plus connue en santé humaine et certains cas iatrogènes de MCJ sont liés à un nettoyage et à une désinfection insuffisants du matériel médical et chirurgical. En effet, une des principales caractéristiques des prions est leur résistance à presque toutes les méthodes classiques d'inactivation. Seuls des traitements drastiques (soude, eau de Javel, autoclavage 134 °C) tels que définis par l'Organisation Mondiale de la Santé (OMS) sont efficaces, mais présentent la plupart du temps des incompatibilités physique ou chimique avec le matériel (WHO, 1999). Dans le cadre du vMCJ, suite à l'exposition de l'homme à l'agent bovin probablement par voie alimentaire, les risques de transmissions iatrogènes à partir de patients en phase d'incubation sont accrus compte-tenu de la présence d'agents infectieux dans les organes périphériques. Il devient donc crucial d'un point de vue de santé publique de pouvoir disposer de procédés d'inactivation efficaces compatibles avec le matériel médico-chirurgical à traiter.

Il existe donc bien un risque avéré de transmission secondaire interhumaine de MCJ via le matériel médico-chirurgical à l'hôpital si celui-ci n'a pas subi des procédures spécifiques (Gibbs *et al*., 1994). En effet, une surface ou un matériel contaminé par l'agent étiologique de ces maladies à prions (PrP) ne peut être traité que selon trois recommandations précises éditées par l'OMS (WHO, 1999) : soit deux procédures chimiques (Javel 20000 ppm au moins 1heure ou soude 1M au moins 1 heure) soit un procédé physique (l'autoclavage à 134°C, 3 bars, 18 minutes). Ces procédures sont drastiques et incompatibles avec les DM, ceux-ci ne peuvent donc être pour le moment qu'incinéré si un cas de MCJ est révélé sur un patient ayant subi une intervention chirurgicale nécessitant des DM. Les dispositifs mis en place à l'hôpital sont très contraignants et sont régis par des circulaires difficiles à appliquer sur le terrain : initialement, c'est la circulaire n°100 (DGS/DHOS du 11 décembre 1995) qui a modifié les réglementations françaises sur les désinfections et les stérilisations hospitalières ainsi que les recommandations à mettre en oeuvre pour sécuriser les actes de biologie et d'anatomie pathologique, en raison du risque ATNC. Quant à la circulaire de 2001 (n°138 DGS/DHOS du 14 mars 2001), elle prend en compte la notion de la vMCJ avec des précautions plus universelles : il est considéré que chaque individu de la population française jusqu'en 2001, même s'il ne présente aucun risque particulier, a pu être exposé à l'agent de l'ESB. Ainsi, contrairement au risque clairement identifié de l'utilisation d'hormones de croissance extractive contaminée, la population n'est pas identifiée pour le risque vMCJ et le principe de précaution s'applique. En revanche, cette circulaire ne prend pas en compte les nouveaux risques, en particulier au niveau de la transfusion sanguine. Si la population exposée au risque de transfusion sanguine était clairement identifiée, on se retrouverait dans le scénario de l'hormone de croissance avec une population à risque identifiée. Les dispositifs mis en place à l'hôpital lors d'une intervention médico-chirurgicale, sont régis selon l'acte et le type de patients traités.

Dans le cadre spécifique des maladies à prions, l'évaluation d'un produit afin qu'il soit listé comme inactivant vis-à-vis des ATNC doit répondre au protocole standard prions (PSP) selon « l'instruction N° DGS/RI3/2011/449 du 1er décembre 2011 relative à l'actualisation des recommandations visant à réduire les risques de transmission d'agents transmissibles non conventionnels lors des actes invasifs. » Ce texte impose l'utilisation d'une tige métallique préalablement contaminée avec du matériel infectieux et exposée à une formulation chimique avant d'être implanté à demeure dans l'animal receveur. Un des problèmes scientifiques majeurs qui se pose est le fait qu'une tige métallique contaminée, exposée et rincée, n'est pas du tout représentative du terrain et qu'il faut s'intéresser au problème de l'inoculum dans son ensemble. A titre d'exemple non restrictif, dans les départements de stérilisation et d'hygiène, au sortir de l'acte chirurgical, les dispositifs médicaux (DM) subissent l'étape de pré-désinfection dans des bains de lavages contenant un produit chimique. Pour autoriser ces effluents à être directement éliminés à l'égout, ce n'est pas l'évaluation d'un produit sur une tige métallique qui permet de répondre à la problématique mais bien un inoculum dans son ensemble.

Aucune solution n'est actuellement proposée pour rendre possible l'inoculation à l'animal receveur (et à fortiori lors de l'utilisation d'une technique *in vitro*) sauf à faire appel à des précipitations chimiques complexes sans être sur du caractère infectieux conservé et des incompatibilités chimiques ou bien à diluer très fortement le produit avant inoculation (Smith *et al*., 2012).

Le document WO 2009/125139 A1 décrit une méthode de détection et/ou de titrage *in vitro* d'un agent transmissible non conventionnel qui est aussi sensible que le bioessai chez l'animal. Il combine à la fois la méthode de titrage appelée « Tissue Culture Infectivity Assay ou TCIA » et un procédé d'amplification appelé « Protein Misfolding Cyclic Amplification ou PMCA ». Cette combinaison consiste à la fois à mettre en contact des dilutions successives d'un homogénat infectieux qui a subi le procédé d'amplification, autrement dit de la conversion d'une forme non pathologique d'ATNC en forme pathologique à partir d'une très petite quantité originelle de protéines pathologiques. Il s'agit d'une approche intéressante de conception d'une méthode de criblage d'un candidat à l'inactivation d'agents pathologiques, celle-ci se limite à un modèle de souche de tremblante non transmissible à l'homme ; un critère qui ne répond donc pas à une problématique de santé humaine. Actuellement seule une méthode *in vivo* validée par les autorités de santé décrite auparavant selon le « protocole standard prions » ou PSP, permet de valider une formulation candidate à l'inactivation des agents pathologiques, basée sur le traitement d'une tige métallique préalablement contaminé avec un homogénat infectieux de la souche 263K et laissée à demeure chez le hamster doré. En résumé, la tige métallique après contamination et traitement subit des bains successifs en eau, s'affranchissant ainsi de toute neutralisation chimique ; considérant que le produit est suffisamment dilué durant ces différentes immersions. C'est pour cela qu'il n'y pas d'injection intracérébrale direct d'un homogénat infectieux traité par une formulation chimique candidate en raison de constituants potentiellement toxiques incompatible avec les données physiologiques.

On peut aussi mentionner les travaux d'une équipe américaine qui a rapporté en 2008 l'inoculation directe d'un homogénat infectieux traité par différentes formulations aux fins de quantifier des protéines prions et déterminer leur niveau de résistance à des traitements d'inactivation (Giles *et al*., 2008).

On peut également mentionner les travaux d'une équipe australienne qui décrivent un procédé de détermination de l'aptitude d'une composition liquide à inactiver un agent transmissible non conventionnel (ATNC) comprenant une détoxication chimique, par neutralisation du pH (Unal et al. Investigation by bioassay of the efficacy of sodium hydroxide treatment on the inactivation of mouse-adapted scrapie. Biologicals 2007, vol. 35 (3), 161-164).

Il existe un besoin pour des procédés améliorés ou alternatifs aux procédés connus, pour l'évaluation des performances de produits destinés à l'inactivation des ATNCs.

### RESUME DE L'INVENTION

La présente invention concerne un procédé de détermination de l'aptitude d'une composition liquide à inactiver un agent transmissible non conventionnel (ATNC) comprenant les étapes suivantes :
a) incuber un ATNC avec une composition à tester,
b) détoxiquer chimiquement et thermiquement le mélange obtenu à la fin de l'étape a), et
c) mesurer l'inactivation dudit ATNC

Dans certains modes de réalisation du procédé, l'étape b) comprend une étape b1) de détoxication chimique du mélange par neutralisation du pH et une étape b2) de détoxication thermique du mélange à une température allant de 60 °C à 95 °C, étant entendu que l'ordre des étapes b1) et b2) est indifférent ; mais il est plus aisé de commencer par la neutralisation suivie du chauffage. Lors de l'approche du point d'ébullition, les études ne sont pas aisées et ne présentent pas d'intérêt supplémentaire pour l'objectif visé.

Dans certains modes de réalisation, l'étape b1) est réalisée par neutralisation du pH à une valeur de pH allant de 6,5 à 8, avantageusement à environ 7,5.

Dans certains modes de réalisation, l'étape b1) est réalisée pendant une durée allant de 10 secondes à 60 minutes, par exemple environ 10 minutes.

Dans certains modes de réalisation, lequel l'étape b2) est réalisée à une température allant de 60 °C à 95 °C, par exemple environ 80 °C,

Dans certains modes de réalisation, lequel l'étape b2) est réalisée pendant une durée allant de 30 secondes à 30 minutes, par exemple environ 1 minute.

Dans certains modes de réalisation, l'ATNC est la forme pathologique de prion PrP^{sc} ou PrP^{res}.

Dans certains modes de réalisation, l'étape c) consiste en un essai *in vitro* de détermination de la présence de l'ATNC, par exemple par une méthode d'immunodétection.

Dans certains modes de réalisation, l'étape c) comprend les étapes suivantes :
c1) administrer le mélange détoxiqué obtenu à la fin de l'étape b) à un mammifère non-humain, et
c2) déterminer la survenue d'une infection dudit mammifère non-humain par ledit ATNC.

Préférentiellement, ledit mélange est administré par voie intra-cérébrale.

Avantageusement, ledit mammifère non-humain est un primate non-humain, préférentiellement un singe, ou un rongeur, préférentiellement choisi parmi un rat, un cobaye, un hamster, un lapin, un campagnol mais surtout une souris.

Avantageusement, la survenue d'une infection dudit mammifère non-humain par ledit ATNC est déterminée par une méthode choisie parmi (i) la détection de signes cliniques associés à une infection par ledit ATNC et (ii) la détection de la présence de l'ATNC dans un échantillon tissulaire provenant dudit mammifère non-humain, par exemple dans un échantillon de tissu cérébral.

### DESCRIPTION DES FIGURES

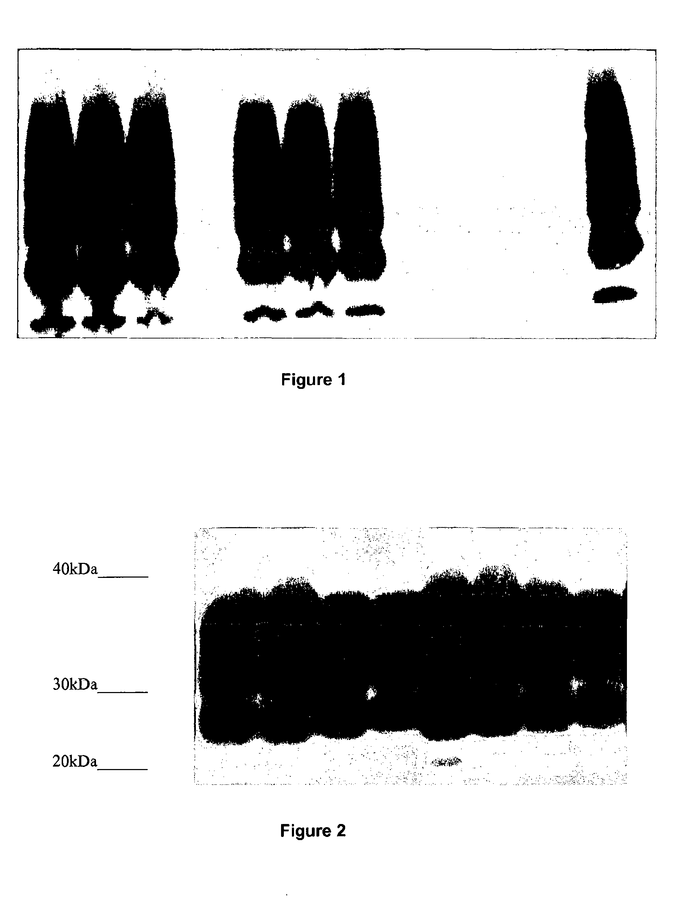

La **Figure 1** est un cliché d'un gel d'électrophorèse polyacrylamide-SDS révélant la migration des protéines prion de la souche 127S responsable de la tremblante du mouton, produites par les cellules RK13 sensibles à la souche de prion 127S. Pistes 1 à 3 : protéines prion produites par les cellules RK13 après inoculation *in vitro* avec une suspension de protéines prion 127S préalablement soumise à la combinaison d'un traitement de détoxication chimique et d'un traitement de détoxication thermique à 80°C. Pistes 4 à 6 : protéines prion produites par les cellules RK13 après inoculation *in vitro* avec une suspension de protéines prion 127S et traitement de détoxication thermique en l'absence de détoxication chimique. Pistes 7 à 9 : témoin négatif: cellules RK13 n'ayant pas été inoculées avec le prion 127S. Piste 10 : témoin positif: protéines prion produites par les cellules RK13 après inoculation *in vitro* avec une suspension de protéines prion 127S qui n'ont pas été soumises à une étape de détoxication chimique ni thermique.
La **Figure 2** est un cliché d'un gel d'électrophorèse polyacrylamide-SDS révélant la migration des protéines prion de la souche vMCJ après amplification *in vitro* selon la méthode PMCA (pour « Protein Misfolding Cyclic Amplification ») et immuno-révélation. Pistes 1 à 4 : protéines prion non traitées, à des dilutions croissantes. Pistes 5 à 8 : protéines prions ayant été soumises au préalable par la combinaison d'un traitement de détoxication par neutralisation du pH et d'un traitement thermique, aux mêmes dilutions que pour les pistes 1 à 4, respectivement.

### DESCRIPTION DETAILLEE DE L'INVENTION

L'invention fournit un procédé pour évaluer le pouvoir inactivant d'une formulation liquide vis-à-vis d'agents infectieux.

On a montré selon l'invention que les inconvénients liés aux procédés connus d'évaluation du pouvoir inactivant d'une composition liquide vis-à-vis d'un ATNC peuvent être surmontés en détoxiquant la composition liquide à tester de manière appropriée pour permettre la réalisation de tests *in vitro* ou *in vivo* de mesure de l'inactivation d'un ATNC en réduisant les biais d'expérience provoqués, ou susceptibles d'être provoqués, par un ou plusieurs constituants de la composition liquide candidate.

Il a été montré selon l'invention que les inconvénients des procédés connus peuvent être surmontés par la réalisation d'une détoxication de la composition liquide à tester, préalablement à la détermination de sa capacité à inactiver un ATNC donné.

Il est montré que, postérieurement à une étape d'incubation d'un ATNC cible avec une composition liquide à tester, la réalisation d'une étape de détoxication de ladite composition liquide rend possible une mesure *in vitro* ou *in vivo* de l'inactivation dudit ATNC sans que l'étape de mesure de l'inactivation dudit ATNC soit affectée, voire soit rendue impossible à exécuter, du fait d'un effet propre de la composition liquide elle-même sur les résultats du test de mesure *in vitro* ou *in vivo* d'inactivation de l'ATNC considéré.

La présente invention est relative à un procédé de détermination de l'aptitude d'une composition liquide à inactiver un agent transmissible non conventionnel (ATNC) comprenant les étapes suivantes :
a) incuber un ATNC avec une composition à tester,
b) détoxiquer chimiquement et thermiquement le mélange obtenu à la fin de l'étape a), et
c) mesurer l'inactivation dudit ATNC

Par « ATNC », au sens de l'invention, on entend un Agent Transmissible Non Conventionnel au sens communément admis par l'homme du métier, ce qui englobe des préparations comprenant ledit ATNC. Un ATNC englobe une forme pathologique d'une protéine prion et des préparations comprenant une forme pathologique d'une protéine prion, tel que le prion PrP^{sc} ou PrP^{res}, ce qui inclut un homogénat infectieux, aussi appelé suspension homogène infectieuse ou inoculât infectieux. On entend par homogénat infectieux ou suspension homogène infectieuse ou inoculât infectieux, la source de protéines infectieuses issues de cerveaux prélevés au stade terminal de la maladie (organe cible de ce type d'infection ou ledit ATNC est le plus concentré ; mais non restrictif). Le cerveau est alors préparé et homogénéisé en solution généralement glucosée avant d'être utilisé en tant que « ATNC ».

L'expression « un ATNC » utilisé à l'étape a) du procédé ci-dessus englobe un ATNC aussi bien qu'une pluralité d'ATNCs.

Dans le cadre de l'invention, le terme "ATNC" représente tout agent transmissible non conventionnel, tels que ceux responsables chez l'être humain de la MCJ familiale ou sporadique, de la maladie du Kuru ou du variant MCJ, ou bien encore ceux responsables chez les animaux d'EST naturelles, telles que la tremblante ovine, l'encéphalopathie spongiforme bovine ou féline, le dépérissement chronique des cervidés ou l'encéphalopathie spongiforme du vison, ou enfin de souches d'EST expérimentalement adaptées à l'animal de laboratoire. On désigne ici par PrP^{sc} ou PrP^{res} la protéine prion de conformation pathologique (ou conformère pathologique). La forme dite " pathologique " de la PrP est la forme de la protéine dont la conformation est corrélée à l'apparition d'une EST chez l'animal humain ou non-humain infectée.

Dans le contexte de l'invention, l'ATNC est également désigné par le terme "agent infectieux ". L'ATNC titré par la méthode selon l'invention est de préférence d'origine ovine, bovine, murine, cricétidienne, cervidée, primate ou humaine. De manière préférentielle, l'ATNC peut être la protéine de conformation pathologique elle-même.

On entend par prions, l'agent infectieux responsable des maladies à prions, ce qui inclut le prion PrP^{sc} ou PrP^{res}.

On entend par agent transmissible non conventionnel ou ATNC, l'agent infectieux prions décrit précédemment au jour de dépôt du brevet; mais en fonction de l'évolution des connaissances, cette liste pourrait être élargie par exemple aux protéines impliquées dans les processus neurodégénératifs comme la protéine Aß (maladie d'Alzheimer), la protéine Huntingtine (maladie de Huntington) et la protéine alpha-synucléine (maladie de Parkinson) entre autres.

Il est montré selon l'invention que l'étape b) de détoxication de la composition liquide préalablement incubée avec l'ATNC, qui est efficace pour réduire ou supprimer les artéfacts de mesure dus aux effets propres de la composition elle-même, n'entraîne aucun effet sur l'ATNC lui-même, et en particulier n'a aucun effet détectable sur ledit ATNC. Il est ainsi montré dans les exemples que l'étape b) de détoxication ne provoque aucun changement structurel détectable de l'ATNC, lequel ATNC (i) migre en électrophorèse selon des bandes protéiques identiques à celles du même ATNC n'ayant pas subi l'étape de détoxication et lequel ATNC (ii) est reconnu par les anticorps se liant à l'ATNC n'ayant pas subi l'étape de détoxication.

Le demandeur pense que les effets propres de compositions liquides candidates pour l'inactivation d'un ATNC dans les tests *in vitro* ou *in vivo* d'inactivation d'ATNC sont générés par leurs propriétés physico-chimiques spécifiques, puisque ces compositions candidates sont formulées de manière à pouvoir détruire la capacité infectieuse de protéines qui sont connues pour être particulièrement résistantes aux traitements physiques et chimiques.

Sans vouloir être lié par une quelconque théorie, le demandeur pense que l'étape b) de détoxication du procédé ci-dessus provoque une réduction ou même une élimination des propriétés délétères de la composition liquide à tester, qui sont génératrices d'artéfacts dans les tests de mesure d'inactivation d'ATNC.

Globalement, il est montré selon l'invention que l'étape b) de détoxication (i) rend possible une mesure fiable et reproductible de l'inactivation d'un ATNC dans un test *in vitro* ou dans un test *in vivo* et (ii) sans affecter l'ATNC cible, du moins de manière détectable.

Avantageusement, l'étape a) d'incubation d'un ATNC avec une composition liquide candidate à tester est réalisée à une température allant depuis la température ambiante pour les DM thermosensibles à moins de 100°C pour les DM thermorésistants, pendant une durée allant de quelques minutes, par exemple 2 minutes, à quelques heures, par exemple 10 minutes, selon le degré de pouvoir inactivant qui est escompté pour la composition candidate liquide à tester.

L'incubation de l'ATNC cible est réalisée en ajoutant la quantité désirée de l'ATNC cible à un volume désiré de la composition candidate à tester, puis en homogénéisant la suspension ainsi obtenue, par exemple par agitation vigoureuse.

Typiquement, une composition liquide candidate peut contenir des éléments alcalins, des enzymes, des agents séquestrants, des tensio-actifs et d'autres excipients, dont la liste n'est pas limitative.

Dans certaines variantes du procédé, dans lesquelles on souhaite mesurer la capacité d'une composition candidate à inactiver une pluralité d'ATNCs cibles, on peut incuber à l'étape a) une pluralité d'ATNCs cibles distincts avec la composition liquide à tester.

Avantageusement, l'étape b) comprend la combinaison d'une étape b1) de détoxication chimique du mélange par neutralisation du pH et d'une étape b2) de détoxication thermique du mélange à une température allant de 60 °C à 95 °C, étant entendu que l'ordre des étapes b1) et b2) est indifférent et que les étapes b1) et b2) peuvent aussi être réalisées simultanément ou quasi-simultanément. ; mais la logique conduit à mener séquentiellement la détoxication chimique suivie de celle thermique.

Avantageusement, l'étape b1) est réalisée par neutralisation du pH à une valeur de pH allant de 6,5 à 8, avantageusement à environ 7,5.

L'homme du métier peut aisément adapter les conditions de l'étape b1) en fonction du pH initial du mélange d'incubation obtenu à la fin de l'étape a). En général, les compositions candidates pour l'inactivation d'un ATNC ont un pH faiblement à fortement alcalin. L'étape b1) consistera donc dans la plupart des cas à ajouter un acide, ou un mélange d'acides, jusqu'à une concentration finale appropriée pour obtenir un pH de la solution allant de 6,5 à 8. Dans de nombreux modes de réalisation de l'étape b1), on ajoute un acide jusqu'à obtenir un pH allant de 7 à 8. Dans certains modes de réalisation, on ajoute un acide jusqu'à obtenir un pH allant de 7,3 à 7,8, par exemple un pH d'environ 7,5.

A l'étape b1) on peut utiliser tout acide dont la neutralisation n'entraine pas un composé toxique mais plutôt facilement assimilable.

A l'étape b2), les conditions du traitement thermique, et en particulier la durée et la température du traitement thermique, sont choisis de manière appropriée par l'homme du métier. Pour détoxiquer des compositions liquides contenant des concentrations finales élevées de tensio-actifs, agents séquestrants, etc., l'homme du métier choisira d'appliquer une température élevée, ou alternativement un traitement thermique de longue durée, ou même à la fois une température élevée et un traitement thermique de longue durée. Dans la pratique, la réalisation de l'étape b2) de traitement thermique à une température allant de 60 °C à 95 °C est appropriée pour détoxiquer les compositions inactivantes candidates préalablement à la mesure de la présence d'ATNC infectieux. Dans certains modes de réalisation de l'étape b2) on applique une température de traitement thermique allant de 75 °C à 85 °C, par exemple une température d'environ 80 °C.

Avantageusement, la durée du traitement thermique de l'étape b2) peut aller de 10 secondes à 60 minutes. L'homme du métier peut aisément adapter la durée de l'étape b2), notamment en fonction de la température de traitement thermique qui a été choisie, et le cas échéant en fonction du type d'ATNC cible que la composition candidate est destinée à inactiver. Pour un ATNC comme la forme pathologique de la protéine prion, qui est très résistant à la chaleur, on peut réaliser l'étape b2) de traitement thermique à des températures élevées, y compris pendant de longues durées, et obtenir une détoxication complète de la composition liquide inactivante candidate sans affecter la capacité infectieuse de l'ATNC cible, et en conséquence sans risque d'altération des résultats de la mesure de l'inactivation de l'ATNC considéré, à l'étape c) du procédé.

A l'étape b), le traitement thermique peut être réalisé selon toute technique connue de l'homme du métier, comme par exemple en utilisant un four à résistances électriques chauffantes ou un dispositif de bain-marie ou bain à sec.

Avantageusement, la durée de l'étape b2) de traitement thermique va de 1 minute à 30 minutes, par exemple de 1 minute à 10 minutes.

Sans vouloir être lié par une quelconque théorie, le demandeur pense que l'étape de traitement thermique constitutive de l'étape b) de détoxication permet d'éliminer certains constituants indésirables susceptibles d'être contenus dans les compositions candidates à tester, et en particulier provoque la destruction des enzymes, provoque la désorganisation du réseau micellaire des agents détergents ou tensio-actifs, et provoque la destruction d'autres ingrédients susceptibles d'induire des changements physiologiques *in vivo.*

Avantageusement, l'ATNC cible est choisi parmi une forme pathologique d'une protéine prion au jour du dépôt du brevet : mais en fonction de l'évolution des connaissances, la liste pourrait par exemple s'étendre à la protéine Aß, la protéine Huntingtine et la protéine alpha-synucléine entre autres.

De préférence, l'ATNC cible est la forme pathologique de prion PrP^{sc} ou PrP^{res}.

Dans certains modes de réalisation du procédé, l'étape c) consiste en un essai *in vitro* de détermination de la présence de l'ATNC, par exemple par une méthode d'immuno-détection. Une méthode d'immuno-détection de ce type est illustrée dans les exemples sous la forme d'un essai d'immuno-empreinte, aussi appelé « Western blot », dans laquelle, après amplification ou non de l'ATNC cible puis migration des protéines amplifiées sur un gel d'électrophorèse, la présence d'une forme infectieuse de l'ATNC cible est révélée à l'aide d'anticorps spécifiques dudit ATNC. L'amplification de l'ATNC, préalable à la migration sur gel d'électrophorèse, est avantageusement réalisée selon la technique PCMA (pour « Protein Misfolding Cyclic Amplification »), telle que décrite par exemple par Saborio et al. (2001) ou encore Gonzalez-Montalban et al. (2011). L'essai d'immuno-détection à l'aide d'anticorps spécifiques de la forme infectieuse de l'ATNC cible (Féraudet *et al*., 2005) peut être réalisé selon la technique décrite par Korth et al. (1997, Nature, Vol. 390 (6655) : 74-77).

A titre illustratif, pour révéler la forme infectieuse de prion PrP^{sc} ou PrP^{res}, on peut utiliser l'anticorps monoclonal CD230 (2G11) commercialisé par la Société Pierce ou encore l'anticorps monoclonal « clone 1.5D7 » commercialisé sous la référence HM5011 par la Société Hycult Biotech. Dans cette variante de réalisation de l'étape c) du procédé, il n'est pas détecté de forme infectieuse de l'ATNC cible lorsque la composition liquide candidate testée est efficace et a complètement inactivé ledit ATNC cible. Les exemples illustrent l'utilisation de l'anticorps Sha31 biotinylé commercialisé sous la référence A03213 par la Société Biorad.

L'étape c) de détection *in vitro* d'une forme infectieuse de l'ATNC cible peut être réalisée également selon une technique d'immuno-détection de type ELISA, comme décrit par exemple dans la demande PCT n° WO 98/37210.

Selon encore une autre variante, l'étape c) de détection *in vitro* d'une forme infectieuse de l'ATNC cible peut être réalisée également selon une technique d'immuno-détection suivant une étape d'amplification de la forme infectieuse de l'ATNC cible par mise en contact d'une solution à tester avec des cellules recombinantes exprimant la forme non pathologique de la protéine considérée, comme décrit par exemple dans les demandes PCT n° WO 2005/022148, WO 2009/125139 et WO 2010/026346, ou comme cela est illustré dans les exemples de la présente demande de brevet.

Les variantes ci-dessus du procédé selon l'invention dans lesquelles l'étape c) est une étape comprenant un essai *in vitro* de détermination de la présence de l'ATNC, par exemple par une méthode d'immuno-détection, présentent les avantages d'être à la fois rapides, fiables et faciles à mettre en oeuvre, et pouvant être réalisées à un coût modéré.

Dans d'autres modes de réalisation du procédé, l'étape c) consiste en un essai d'infectiosité *in vivo* et l'étape c) comprend les étapes suivantes :
c1) administrer le mélange détoxiqué obtenu à la fin de l'étape b) à un mammifère non-humain, et
c2) déterminer la survenue d'une infection dudit mammifère non-humain par ledit ATNC.

Avantageusement, dans ces autres modes de réalisation du procédé ledit mélange est administré à l'étape c1) par une voie choisie parmi les voies sous-cutanée, intra-dermique, intra-péritonéale, intra-veineuse ou intra-cérébrale entre autres. De manière tout à fait préférée, ledit mélange est administré par voie intra-cérébrale.

Selon une variante préférée de ces autres modes de réalisation du procédé, ledit mammifère non-humain est sacrifié à la fin de l'étape c). Ainsi, selon cette cariante préférée, ledit mammifère non-humain est sacrifié à une étape c3) qui suit l'étape c2) de détermination de la survenue d'une infection dudit mammifère non-humain par ledit ATNC.

Dans ces autres modes de réalisation du procédé, ledit mammifère non-humain est avantageusement (i) un primate non-humain, préférentiellement un singe, ou bien (ii) un rongeur, préférentiellement choisi parmi un rat, un cobaye, un hamster, un lapin, un campagnol ou encore une souris.

Dans ces autres modes de réalisation du procédé, on peut utiliser un mammifère non-humain (généralement une souris) exprimant un transgène codant une forme non pathologique de l'ATNC cible, par exemple mammifère non-humain exprimant un transgène codant la forme non pathologique de la protéine prion humaine (PrP). On peut notamment utiliser les souris transgéniques pour le gène codant la PrP humaine telles que celles décrites par Beringue *et al*. (2008) ou encore celles décrites dans les documents de brevet US 5,792,901, US 5,908,969, US 6,008,435 ou WO 97/04814.

Dans ces autres modes de réalisation du procédé, la survenue d'une infection dudit mammifère non-humain par ledit ATNC est déterminée par une méthode choisie parmi (i) la détection de signes cliniques associés à une infection par ledit ATNC et (ii) la détection de la présence de l'ATNC dans un échantillon tissulaire provenant dudit mammifère non-humain, par exemple dans un échantillon de tissu cérébral.

Classiquement, les signes cliniques associés à une infection par un ATNC, en particulier une forme pathologique d'une protéine prion, y compris la protéine PrP^{sc} ou PrP^{res}, sont les suivants :
- troubles du comportement, notamment excitabilité, troubles de la nidation, troubles du toilettage,
- perte du réflexe de positionnement visuel,
- ataxie cérébelleuse, démarche anormale, prostration,
- obésité puis amaigrissement,
- paraplégie, puis tétraplégie.

Les critères de diagnostic clinique associés à une infection par un ATNC chez la souris sont rapportés notamment par Carlson *et al*. (1986).

Dans ces autres modes de réalisation du procédé, la détection de l'ATNC dans un échantillon tissulaire, en particulier dans un échantillon de cerveau, peut être réalisée comme décrite par exemple dans la demande PCT n° WO 02/059615, en particulier les exemples 2 et 3 de ce document.

La variante ci-dessus de réalisation du procédé de l'invention dans laquelle l'étape c) comprend la détermination *in vivo* de la survenue d'une infection dudit mammifère non-humain par ledit ATNC présente l'avantage de mimer les conditions physiologiques d'infection d'un mammifère, y compris l'homme, par un ATNC, bien que cette variante soit par ailleurs plus longue et plus coûteuse que les variantes de mesure de l'inactivation d'ATNC cibles réalisée *in vitro* décrites précédemment dans la présente description.

Il ressort de l'ensemble de la description que le procédé de l'invention permet le criblage de compositions liquides candidates (ou sous forme de poudre à reconstituer extemporanément) pour leur aptitude à inactiver un ATNC cible, y compris une forme pathologique de prion comme la protéine PrP^{sc}.

Notamment, le procédé selon l'invention, du fait de l'étape b) de détoxication de la composition candidate, rend possible une mesure *in vivo* de la présence résiduelle d'ATNC ayant conservé un pouvoir infectieux, dans des conditions physiologiques, au contraire des procédés connus qui comprennent des conditions non-physiologiques d'insertion d'une tige métallique dans le cerveau

Le procédé selon l'invention apporte notamment les avantages suivants :
- La possibilité de neutraliser l'action d'un détergent prionicide, suivie du chauffage,
- De rendre l'inocuité du mélange détergent prionicide+neutralisant et chauffage,
- De vérifier par une neutralisation immédiate au préalable de la mise en contact avec la source infectieuse que le caractère infectieux de la protéine est bien conservé (par bioessai à l'animal receveur) ; on vérifie par une technique *in vitro* d'amplification (PMCA) la conservation de la signature caractéristique de la protéine (technique aussi sensible que le bioessai),
- Dans le cadre des maladies à prions, cette technique est revendiquée pour toute source de protéines infectieuses aussi bien humaine qu'animal ; de même, elle peut être élargie aux protéines recombinantes et synthétiques,
- De tels essais sont menés pour prouver *in fine* le pouvoir prionicide pour une application aux dispositifs médicaux (DM),
- Pour le moment les autres protéines impliquées dans les processus neurodégénératifs ne sont pas considérées comme transmissibles dans les conditions standards ; mais cette technique pourra également être utilisée, le principe restant le même,
- Pour l'étape de chauffage, celle-ci est menée à une température donnée pour un temps donné ; le protocole étant ajusté en fonction du type de protéine et du détergent. Par exemple pour les prions, le chauffage est à 80 °C pendant une minute,

La technique proposée ici comprenant une étape de détoxication chimique et thermique d'une composition candidate est particulièrement destinée à évaluer par des techniques *in vitro* et *in vivo* toute formulation en solution candidate à l'inactivation d'agents infectieux en solution.

Le domaine d'application du procédé selon l'invention est très large puisqu'il permet de tester ou cribler des produits candidates utiles depuis le laboratoire de recherche, jusqu'au nettoyage de locaux suspectés d'être contaminés, toute sorte de matériel ou surface en contact avec ces agents infectieux comme des camions de transports.

Parmi les nombreux agents impliqués dans les processus neurodégénératifs, seuls les prions à ce jour sont transmissibles dans les conditions opératoires normales. C'est pourquoi le procédé selon l'invention vise en premier lieu l'inactivation des prions mais ne se limite pas à ces seuls agents pathogènes. Le procédé de l'invention pourrait s'appliquer aussi à l'inactivation de tout type d'agent impliqué dans un processus dégénératif, en fonction de l'évolution des connaissances et sous réserve que l'agent identifié soit bien résistant à la température requise lors de l'étape de chauffage suivant la neutralisation par le neutralisant.

La présente invention est en outre illustrée par les exemples ci-après.

### Exemple 1: Procédé in vivo de détermination du pouvoir inactivant d'une composition vis-à-vis d'un agent transmissible non conventionnel (ATNC)

### A. Matériels et Méthodes

### A.1. Compositions candidates à tester

Deux compositions candidates ont été testées pour leur aptitude à inactiver un ATNC, et plus particulièrement une forme pathologique d'une protéine prion ; mais aussi pour valider le procédé de neutralisation chimique et thermique revendiqué.

Pour une des formules candidates, celle-ci contient un sel alcalin, un tensio-actif non ionique, un tensio-actif amphotère et un séquestrant; utilisée à 0.8 % dont la concentration finale de chacun des composants est la suivante :
- potasse caustique écaillée : 0.376 %
- tensio-actif non ionique : 0.080 %
- tensio-actif amphotère (à 70 %) : 0.026 %
- séquestrant : 0.012 %

Pour une autre des formules candidates, celle-ci contient des sels alcalins, un ammonium quaternaire, des enzymes et un anti-corrosif; utilisé à 1 % dont la concentration finale de chacun des composants est la suivante :
- sels de soude 0.4670 %
- potasse caustique écaillé : 0.4894 %
- ammonium quaternaire : 0.0092 %
- enzymes : 0.0330 %
- benzotriazole : 0.0010 %
Le traitement contrôle de référence recommandée par l'OMS reste la soude à 1M et constitue notre contrôle interne d'expérience.

### A.3. Source d'ATNC

Comme source d'ATNC nous avons choisi des souris transgéniques humanisées tg650 exprimant la PrP humaine et infectées avec la forme sporadique la plus répandue chez l'homme appelée MM1 (Beringue *et al*., 2008, supplementary data) au stade terminale de la maladie. Le procédé peut bien entendu s'appliquer à toute source d'ATNC.

### A.2. Animaux

Les souris décrites dans le paragraphe précédent ont été inoculées à l'âge de 8 semaines et selon le protocole décrit dans le tableau ci-après (modèle publié et propriété de l'INRA). L'étude est menée dans une animalerie accréditée de niveau 3 régie selon les textes en vigueur en particulier au niveau des conditions d'hébergement, et d'éthique entre autres.

La formulation candidate codée C dans le tableau ci-dessous est utilisée à 0.8 % pendant 10 minutes à 55 °C.

L'autre formulation candidate codée E dans le tableau ci-dessous est utilisée à 1 % pendant 15 minutes à 20 °C.

La soude est utilisée à 1M pendant 1 heure à 20 °C.

| **Inoculum en direct** | | |
|---|---|---|
| **Traitements** | **Code** | **Nombre** |
| Formulation candidate | C | 6 |
| Formulation candidate | E | 6 |
| Référence soudé | S | 4 |
| | | |

| **Gamme contrôle** | | |
|---|---|---|
| 10⁻² | P2 | 6 |
| 10⁻³ | P3 | 6 |
| 10⁻⁴ | P4 | 6 |
| 10⁻⁵ | P5 | 6 |
| 10⁻⁶ | P6 | 6 |
| 10⁻⁷ | P7 | 6 |
| 10⁻⁸ | P8 | 5 |
| | | |
| **Point contrôle** Formulation candidate neutralisée avant mise en contact avec l'inouclum 10⁻² | PC2 | 6 |

### A.2. Conditions de détoxication chimique et thermique

Pour le produit de référence chimique, la soude, les conditions de détoxication ont été pour un volume de produit l'addition d'un volume de neutralisant, et pour les deux formulations candidates, de même, à un volume de produit à été rajouté un volume de neutralisant. Enfin pour le traitement de référence et les deux formulations candidates, après l'addition du neutralisant, un chauffage à 80 °C pendant 1 minute.

### A.3. Mesure in vivo de l'infectiosité

L'infectiosité résiduelle éventuelle des compositions liquides candidates a été évaluée par l'observation de la survenue de signes cliniques associés à la présence d'ATNC infectieux, comme décrit par exemple par Fichet *et al.* en 2004.

### A.4. Format du test

Des tests *in vivo* au préalable d'une étude avec un homogénat infectieux ont été réalisés selon le protocole ci-dessous (sans agents infectieux ; étude de toxicité).
- ***deux*** produits (formulations candidates inactivantes vis-à-vis du prion citées précédemment) ont été détoxiqués par neutralisation du pH et traitement thermique,
- le mélange résultant a ensuite été dilué, volume à volume, dans une solution glucosée, afin d'obtenir une suspension de test glucosée,
- un volume de 10 µL de la suspension de test glucosée a ensuite été inoculé par voie intracérébrale dans l'animal receveur (groupe de 6 animaux par groupe de dilution),
- l'étude a été menée pendant 28 jours selon les conditions suivantes :

**Tableau 1 : conditions du test de toxicité (sans agent infectieux)**

| | Sans Formulation | Avec Formulation |
|---|---|---|
| Sans Neutralisant et sans Chauffage | **Condition non létale (contrôle)** | **Condition létale** |
| Avec Neutralisant sans Chauffage | **Condition létale** | **Condition létale** |
| Sans Neutralisant avec Chauffage | **Condition non létale (contrôle)** | **Condition létale** |
| Avec Neutralisant et Chauffage | **Condition létale** | **Condition non létale** |

Les résultats montrent que la suspension de test glucosée ne présente aucune toxicité pour les animaux, ce qui illustre que l'étape de détoxication par neutralisation du pH et traitement thermique est efficace, et va permettre d'éviter la survenue d'artéfacts expérimentaux *in vivo* pour la mesure de la présence éventuelle d'un ATNC infectieux dans la solution de test glucosée, et donc dans le mélange composition liquide candidate + ATNC testé.

L'absence de toxicité du mélange détoxiqué a rendu possible la réalisation d'une étude à grande échelle avec les agents pathogènes, selon le protocole suivant : chaque produit candidat (composition liquide inactivante candidate) a été préalablement détoxiqué par neutralisation du pH et traitement thermique avant d'être mélangé avec une suspension homogène contenant les agents infectieux en solution puis inoculé directement par voie intracérébrale à l'animal receveur.

Le témoin d'innocuité de l'étape de détoxication est l'inoculation intra-cérébrale de la suspension homogène contenant seulement les agents infectieux en solution.

La validation du procédé revendiqué ici correspond au mélange au préalable d'une formulation candidate (limitée à une seule pour minimiser le nombre d'animaux utilisés) avec la solution neutralisante avant la mise en contact avec la source de prion, suivie de l'étape de chauffage avant l'inoculation.

Les résultats obtenus seront comparés en terme d'incubation de la maladie et du profil du marqueur pathognomonique de ces maladies.

### B. Résultats de la mesure de l'infectiosité résiduelle et validation du procédé de neutralisation

Pour les compositions candidates testées sur le modèle tg650/MM1, aucun signe clinique associé à une infection par l'ATNC cible n'a été observé au jour de dépôt de la présente invention. La première version proposée ici sera complétée une fois les résultats connus.

### Exemple 2 : Test in vitro d'inactivation d'un ATNC sur des cellules sensibles au prion pathogène

A l'exemple 2, on a utilisé la technique de dosage d'ATNC *in vitro* sur des cellules qui est décrite dans le demande PCT n° WO 2009/125139.

Les cellules RK13 sensibles aux prions de la tremblante 127S ont été utilisées. Les cellules ont été cultivées selon un protocole standard dans une plaque 6 puits à raison de 200000 cellules par puits. Les cellules ont été ensuite mises en contact avec l'inoculum selon le protocole suivant :
- un traitement chimique a été préalablement neutralisé par notre solution neutralisante avant la mise en contact avec l'inoculum puis chauffé 1 minute à 80 °C (car ce sont des prions) avant mise en contact des cellules (pistes 1, 2 et 3),
- le traitement chimique et la solution neutralisante ont été remplacés par de l'eau pour valider le protocole de neutralisation chimique (pistes 4, 5 et 6),
- le contrôle cellules seules est représenté des pistes 7 à 9,
- le contrôle de référence d'infection des cellules (inoculum 127S) est représenté piste 10

Les cellules ont été passées une fois afin d'éliminer l'inoculum résiduel. Les culots cellulaires ont ensuite été récupérés en vue de la détection du marqueur prion par la technique de Westem-Blot.

En résumé des cellules sensibles à l'infection par des agents pathogènes ont été choisies. Des formulations candidates ont été préalablement neutralisés par le neutralisant et soumis au chauffage avant la mise en contact avec les agents infectieux, puis exposées ensuite aux cellules susceptibles. Ce protocole a été comparé aux agents infectieux directement mis en contact avec les cellules. Les résultats de l'Exemple 2 montrent qu'il n'y a pas de différence dans l'infection des cellules entre les deux conditions décrites, validant ainsi notre procédé de neutralisation dans le cadre de l'utilisation d'un modèle cellulaire comme modèle complémentaire de criblage pour retenir une formulation candidate à l'inactivation d'agents pathogènes.

Les résultats de la Figure 1 montrent que le signal caractéristique de la protéine PrPres est parfaitement conservé après application de l'invention, prouvant ainsi que le procédé de neutralisation chimique associée au chauffage ne change pas la migration et la détection de celle-ci.

### Exemple 3 : Test in vitro d'inactivation d'un ATNC

Amplification du signal par PMCA et détection du marqueur pathognomonique par immuno-empreinte (« Western-Blot ») : tout comme pour le modèle cellulaire décrit à l'exemple 2.

Des compositions candidates ont été préalablement détoxiquées par neutralisation du pH autour de 7.5 et traitement chimique à 80 °C pendant 1 minute avant la mise en contact avec les agents infectieux. Ce protocole a été comparé aux agents infectieux soumis directement à amplification. Les résultats de l'Exemple 3 montrent qu'il n'y a aucune différence de signature de la protéine pathologique entre les deux conditions décrites, validant ainsi le procédé de détoxication, lequel n'affecte pas l'ATNC.

Des prions contenus dans une suspension homogène ont été :
- soit directement utilisés pour être amplifiés. Sur la Figure 2, les pistes 1 à 4 correspondent à une série de dilutions croissantes de prion ; piste 1 : 40 µg équivalent cerveau ; piste 2 : 4 µg équivalent cerveau ; piste 3 : 0,4 µg équivalent cerveau ; piste 4 : 0,04 µg équivalent cerveau ;
- soit la composition liquide candidate a été mélangée avec une suspension homogène d'agents infectieux, puis détoxiquée par neutralisation du pH et traitement thermique. Sur la Figure 2, les pistes 5 à 8 correspondent à une série de dilutions croissantes de prion ; piste 1 : 40 µg équivalent cerveau ; piste 2 : 4 µg équivalent cerveau ; piste 3 : 0,4 µg équivalent cerveau ; piste 4 : 0,04 µg équivalent cerveau ;

Le produit après amplification a été traité par un traitement protéolytique à la protéinase K, dénaturé et déposé sur électrophorèse en gel de polyacrylamide (SDS Bis-Tris 12%).

Après migration, les protéines ont été transférées sur une membrane de nitrocellulose puis immuno-révélées par un premier anticorps (Sha31 biotinylé, Biorad) puis par de la streptavidine couplée à une peroxydase (Pierce).

Les résultats sont présentés sur la Figure 2. Les protéines sont mises en évidence par chimiluminescence et autoradiographie. La signature caractéristique de la protéine infectieuse est observée par 3 bandes correspondant aux 3 isoformes de la protéine (non, mono et bi-glycosylé).

### REFERENCES BIBLIOGRAPHIQUES :

Beringue V, Le Dur A, Tixador P, Reine F, Lepourry L, Perret-Liaudet A, Haik S, Vilotte JL, Fontes M, Laude H. Prominent and Persistent Extraneural Infection in Human PrP Transgenic Mice Infected with Variant CJD. PLoS ONE 2008 (1)
Carlson GA, Kingsbury DT, Goodman PA, Coleman S, Marshall ST, DeArmond S, Westaway D, and Prusiner SB. Linkage of prion protein and scrapie incubation time genes. Cell, Volume 46, Issue 4, 503-511, 15 August 1986.
Circulaire N°DGS/SD5C/DHOS/2005/435 du 23 septembre 2005 relative aux recommandations pour le traitement des dispositifs médicaux utilisés chez les sujets ayant reçu des produits sanguins labiles (PSL) provenant de donneurs rétrospectivement atteints de variant de la maladie de Creutzfeldt-Jakob (vMCJ)
Circulaire N°DGS/DH N°100 du 11 décembre 1995 relative aux précautions à observer en milieu chirurgical et anatomo-pathologique face aux risques de transmission de la maladie de Creutzfeldt-Jakob.
Féraudet C, Morel N, Simon S, Volland H, Frobert Y, Créminon C, Vilette D, Lehmann S, and Grassi J. Screening of 145 Anti-PrP Monoclonal Antibodies for Their Capacity to Inhibit PrPSc Replication in Infected Cells. JBC Papers in Press, December 23, 2004.
Fichet G, Comoy E, Duval C, Antloga K, Dehen C, Charbonnier A, McDonnell G, Brown P, Lasmézas CI, Deslys JP. Novel methods for disinfection of prion-contaminated medical devices. Lancet 2004;364:521-6.
Ghani AC, Donnelly CA, Ferguson NM and Anderson RM. Updated projections of future vCJD deaths in the UK. BMC Infectious Diseases 2003, 3:4.
Gibbs CJ, Jr., Asher DM, Kobrine A, Amyx HL, Sulima MP, Gajdusek DC. Transmission of Creutzfeldt-Jakob disease to a chimpanzee by electrodes contaminated during neurosurgery. J Neurol Neurosurg Psychiatry 1994;57(6):757-8.
Giles K, Glidden D, Beckwith R, Seoanes R, Peretz D, Stephen J. DeArmond S, Prusiner S. Resistance of Bovine Spongiform Encephalopathy (BSE) Prions to Inactivation. PloS Pathogens 2008 4(11).
Gonzalez-Montalban N, Makarava N, Ostapchenko V, Savtchenk R, Alexeeva I, Rohwer R, Baskakov I. Highly Efficient Protein Misfolding Cyclic Amplification. PLoS Pathogens 2011 7(2).
Instruction N° DGS/RI3/2011/449 du 1er décembre 2011 relative à l'actualisation des recommandations visant à réduire les risques de transmission d'agents transmissibles non conventionnels lors des actes invasifs. Validée par le CNP le 1er décembre 2011 - Visa CNP 2011-297.
Korth C, Stierli B, Streit P, Moser M, Schaller O, Fischer R, Schulz-Schaeffer W, Kretzschmar H, Raeberk A, BraunU, Ehrensperger F, Hornemann S, Glockshuber R, Riek R, Billeter M, Wuthrich K & Oesch B. Prion (PrPSc)-specific epitope defined by amonoclonal antibody. Nature, vol 390, 6 November 1997.
Smith JD, Greenlee JJ, Foster GH, and Nicholson EM. Acetone Precipitation of the Scrapie Agent Results in Successful Recovery of PrPsc but Decreased Infectivity. J. Agric. Food Chem. 2012, 60, 4758-4762.
Soto C, Saborio G and Anderes L. Cyclic amplification of protein misfolding: application to prion-related disorders and beyond. Trends in Neurosciences 2002 25(8).
WHO Infection control guidelines for transmissible spongiform encephalopathies. World Health Organization report 23-26 march 1999, WHO/CDS/CSR/APH/2000.3, 1999.

## Revendications

1. Procédé de détermination de l'aptitude d'une composition liquide à inactiver un agent transmissible non conventionnel (ATNC) comprenant les étapes suivantes :
a) incuber un ATNC avec une composition à tester,
b) détoxiquer chimiquement et thermiquement le mélange obtenu à la fin de l'étape a), et
c) mesurer l'inactivation dudit ATNC afin d'évaluer le pouvoir en activant de ladite composition.

2. Procédé selon la revendication 1, dans lequel l'étape b) comprend une étape b1) de détoxication chimique du mélange par neutralisation du pH et une étape b2) de détoxication thermique du mélange à une température allant de 60 °C à 95 °C, étant entendu que l'ordre des étapes b1) et b2) est indifférent.

3. Procédé selon la revendication 2, dans lequel l'étape b1) est réalisée par neutralisation du pH à une valeur de pH allant de 6,5 à 8, avantageusement à environ 7,5.

4. Procédé selon l'une des revendications 2 et 3, dans lequel l'étape b1) est réalisée pendant une durée allant de 10 secondes à 60 minutes, par exemple environ 10 minutes.

5. Procédé selon l'une des revendications 2 à 4, dans lequel l'étape b2) est réalisée à une température allant de 60 °C à 95 °C, par exemple environ 80 °C.

6. Procédé selon l'une des revendications 2 à 5, dans lequel l'étape b2) est réalisée pendant une durée allant de 30 secondes à 30 minutes, par exemple environ 1 minute.

7. Procédé selon l'une des revendications 1 à 6, dans lequel l'ATNC est choisi parmi une forme pathologique d'une protéine prion, la protéine Aß, la protéine Huntingtine et la protéine alpha-synucléine.

8. Procédé selon l'une des revendications 1 à 7, dans lequel l'ATNC est la forme pathologique de prion PrP^{sc} ou PrP^{res}.

9. Procédé selon l'une des revendications 1 à 8, dans lequel l'étape c) consiste en un essai *in vitro* de détermination de la présence de l'ATNC, par exemple par une méthode d'immunodétection.

10. Procédé selon l'une des revendications 1 à 8, dans lequel l'étape c) comprend les étapes suivantes :
c1) administrer le mélange détoxiqué obtenu à la fin de l'étape b) à un mammifère non-humain, et
c2) déterminer la survenue d'une infection dudit mammifère non-humain par ledit ATNC.

11. Procédé selon la revendication 10, dans lequel, à l'étape c1), ledit mélange est administré par voie sous-cutanée, intra-dermique, intra-péritonéale, intra-veineuse ou d'autre voie compatible ou préférentiellement intra-cérébrale.

12. Procédé selon l'une des revendications 10 et 11, dans lequel ledit mammifère non-humain est un primate non-humain, préférentiellement un singe, ou un rongeur, préférentiellement choisi parmi un rat, un cobaye, un hamster, un lapin, un campagnol, une souris entre autres.

13. Procédé selon l'une des revendications 10 à 12, dans lequel la survenue d'une infection dudit mammifère non-humain par ledit ATNC est déterminée par une méthode choisie parmi (i) la détection de signes cliniques associés à une infection par ledit ATNC et (ii) la détection de la présence de l'ATNC dans un échantillon tissulaire provenant dudit mammifère non-humain, par exemple dans un échantillon de tissu cérébral.

## Patentansprüche

1. Verfahren zum Bestimmen der Fähigkeit einer flüssigen Zusammensetzung, eine unkonventionelle übertragbare Substanz (ATNC) zu inaktivieren, mit folgenden Schritten:
a) Inkubieren einer ATNC mit einer zu prüfenden Zusammensetzung,
b) chemisches und thermisches Entgiften der am Ende von Schritt a) erhaltenen Mischung, und
c) Messen der Inaktivierung der ATNC zum Bewerten der Inaktivierungskraft der Zusammensetzung.

2. Verfahren nach Anspruch 1, in dem der Schritt b) einen Schritt b1) zum chemischen Entgiften der Mischung durch pH-Neutralisieren und einen Schritt b2) zum thermischen Entgiften der Mischung bei einer Temperatur von 60°C bis 95°C umfasst, wobei die Reihenfolge der Schritte b1) und b2) gleichgültig ist.

3. Verfahren nach Anspruch 2, in dem der Schritt b1) durch pH-Neutralisieren auf einen pH-Wert von 6,5 bis 8, vorzugsweise etwas 7,5 erfolgt.

4. Verfahren nach einem der Ansprüche 2 und 3, in dem der Schritt b1) über eine Dauer von 10 Sekunden bis 60 Minuten, beispielsweise etwa 10 Minuten erfolgt.

5. Verfahren nach einem der Ansprüche 2 bis 4, in dem der Schritt b2) bei einer Temperatur von 60°C bis 95°C, beispielsweise etwa 80°C erfolgt.

6. Verfahren nach einem der Ansprüche 2 bis 5, in dem der Schritt b2) über eine Dauer von 30 Sekunden bis 30 Minuten, beispielsweise etwa einer Minute erfolgt.

7. Verfahren nach einem der Ansprüche 1 bis 6, in dem die ATNC aus einer pathologischen Form eines Prionenproteins, dem Aβ-Protein, dem Hungtingtin-Protein und dem α-Synuklein-Protein ausgewählt ist.

8. Verfahren nach einem der Ansprüche 1 bis 7, in dem die ATNC die pathologische Prionenform PrP^{sc} oder PrP^{res} ist.

9. Verfahren nach einem der Ansprüche 1 bis 8, in dem der Schritt c) ein *in vitro* Essay zur Bestimmung des Vorliegens der ATNC, beispielsweise mittels eines Immunnachweisverfahrens aufweist.

10. Verfahren nach einem der Ansprüche 1 bis 8, in dem der Schritt c) folgende Schritte umfasst:
c1) Verabreichen der am Ende des Schritts b) gewonnenen entgifteten Mischung an einen nicht menschlichen Säuger, und
c2) Bestimmen des Auftretens einer Infektion des nicht menschlichen Säugers durch die ATNC.

11. Verfahren nach Anspruch 10, in dem bei Schritt c1) die Mischung durch einen subkutanen, intradermalen, intraperitonealen, intravenösen oder anderen kompatiblen oder vorzugsweise intrazerebralen Weg verabreicht wird.

12. Verfahren nach einem der Ansprüche 10 bis 11, in dem der nicht menschliche Säuger ein nicht menschlicher Primat, vorzugsweise ein Affe, oder ein Nagetier, vorzugsweise ausgewählt unter anderem unter einer Ratte, einem Meerschweinchen, einem Hamster, einem Kaninchen, einer Wühlmaus bzw. einer Maus, ist.

13. Verfahren nach einem der Ansprüche 10 bis 12, in dem das Auftreten einer Infektion des nicht menschlichen Säugers durch die ATNC durch ein Verfahren bestimmt wird, das aus (i) dem Nachweis klinischer Symptome für eine Infektion durch die ATNC und (ii) dem Nachweis des Vorliegens der ATNC in einer Gewebeprobe aus dem nicht menschlichen Säuger, beispielsweise in einer zerebralen Gewebeprobe ausgewählt ist.

## Claims

1. Method for determining the ability of a liquid composition to inactivate a non-conventional transmissible agent (NCTA), comprising the following steps:
a) incubating an NCTA with a composition to be tested,
b) chemically and thermally detoxifying the mixture obtained at the end of step a), and
c) measuring the inactivation of said NCTA in order to evaluate the inactivating power of said composition.

2. Method according to claim 1, wherein step b) comprises a step b1) for the chemical detoxification of the mixture by pH neutralisation and a step b2) for the thermal detoxification of the mixture at a temperature from 60°C to 95°C, on the understanding that the order of steps b1) and b2) is irrelevant.

3. Method according to claim 2, wherein step b1) is carried out by neutralising the pH to a pH value of from 6.5 to 8, advantageous to approximately 7.5.

4. Method according to one of claims 2 and 3, wherein step b1) is carried out for a duration of from 10 seconds to 60 minutes, for example approximately 10 minutes.

5. Method according to one of claims 2 to 4, wherein step b2) is carried out at a temperature of from 60°C to 95°C, for example approximately 80°C.

6. Method according to one of claims 2 to 5, wherein step b2) is carried out for a duration of from 30 seconds to 30 minutes, for example approximately 1 minute.

7. Method according to one of claims 1 to 6, wherein the NCTA is selected from a pathological form of a prion protein, the Aβ protein, the Huntingtin protein and the alpha-synuclein protein.

8. Method according to one of claims 1 to 7, wherein the NCTA is the pathological form of the PrP^{sc} or PrP^{res} prion.

9. Method according to one of claims 1 to 8, wherein step c) consists of an *in vitro* test for determining the presence of the NCTA, for example by an immunodetection method.

10. Method according to one of claims 1 to 8, wherein step c) comprises the following steps:
c1) administering the detoxified mixture obtained at the end of step b) to a non-human mammal, and
c2) determining the occurrence of an infection in said non-human mammal with said NCTA.

11. Method according to claim 10, wherein, in step c1), said mixture is administered by a subcutaneous, intradermal, intraperitoneal or intravenous route or another compatible or, preferably, intracerebral, route.

12. Method according to one of claims 10 and 11, wherein said non-human mammal is a non-human primate, preferably an ape, or a rodent, preferably selected from a rat, a guinea pig, a hamster, a rabbit, a vole or a mouse, among others.

13. Method according to one of claims 10 to 12, wherein the occurrence of an infection in said non-human mammal with said NCTA is determined by a method chosen from (i) the detection of clinical signs associated with an infection by said NCTA and (ii) the detection of the presence of the NCTA in a tissue sample from said non-human mammal, for example in a cerebral tissue sample.
